# EUROPEAN PATENT APPLICATION

(11) **EP 1 685 824 A1**
(43) Date of publication of application: **02.08.2006**
(21) Application number: 06100458.6
(22) Date of filing: 17.01.2006
(51) Int. Cl.: A61K 8/25, A61K 8/19, A61Q 19/06, A61Q 19/08

(54) **Cosmetic composition**

(30) Priority: 30.01.2005 IL 16664605
(71) Applicant: Or-Le-Or Ltd., Haifa 35414 (IL)
(72) Inventor: Ingman, Dov, 34366, Haifa (IL)
(74) Representative: Schmitz, Jean-Marie

(57) **Abstract**

The present invention relates to a new topical cosmetic composition formulated for concealing wrinkles and for eliminating or reducing damages to the skin appearance resulted from a wide variety of disorders, such as for example, acne. Said composition comprises water, optionally containing 25 - 400 ppm of Ag, hydrophobic particles, preferably hydrophobic silica, having a diameter, ranged from about 5 to about 150nm, and/or hydrophilic particles, preferably hydrophilic silica, having a diameter, ranged from about 5 to about 150 nm and a soluble electrolyte, capable of releasing free ions in an aqueous environment.

## Description

### Field of the invention

The present invention relates to a composition comprising water, optionally containing 25 - 400ppm of Ag, hydrophobic particles, preferably having a diameter, ranged from about 5 to about 150 nm, and/or hydrophilic particles preferably having a diameter, ranged from about 5 to about 150 nm and a soluble electrolytic component, capable of releasing free ions in an aqueous environment, referred herein to as an electrolyte, wherein when the concentration of hydrophilic particles is 0wt% then the concentration of hydrophobic particles is >0wt% and when the concentration of hydrophobic particles is 0wt% then the concentration of hydrophilic particles is >0wt%. When the hydrophobic particles are not present in the composition, it is in a colloid-gel form and when the hydrophilic particles are not present in the composition, it is in a colloid-powder form in which the hydrophobic particles bound shells that encapsulate aqueous solution droplets. When hydrophilic and hydrophobic particles are present in the composition it simultaneously comprises bounded shells encapsulating a gaseous material, dispersed in water and bounded shells encapsulating aqueous solution droplets. Such composition, according to the present invention, that presents a characteristic emulsion structure (namely, the presence of bounded shells encapsulating gas, coated by an external layer of hydrophilic particles and an internal layer of hydrophobic particles), side by side, with a characteristic powder structure (namely, the presence of bounded shells encapsulating aqueous solution droplets, coated by an external layer of hydrophobic particles and an internal layer of hydrophilic particles), referred herein to as a bi-phase composition.
More specifically, a bi-phase composition, according to the present invention, comprises a certain proportion of bounded shells encapsulating gas dispersed in electrolytic solution, herein referred to as an emulsion phase and bounded shells encapsulating aqueous solution droplets, herein referred to as a powder phase. It should be pointed out that the presence of electrolyte in the composition of present invention plays a key role in establishing said unique bi-phase state.
The composition of present invention represents two forms of powder phases:
(a) The powder phase in a bi-phase composition which comprises water, hydrophobic particles, hydrophilic particles and an electrolytic component is represented by a bounded shell encapsulating aqueous solution droplets, coated by an external layer of hydrophobic particles and an internal layer of hydrophilic particles; and
(b) The powder phase in a composition which comprises water, hydrophobic particles and an electrolytic component is represented by a bounded shell encapsulating aqueous solution droplets, coated by an external layer of hydrophobic particles.

The present invention further relates to a topical cosmetic composition formulated for concealing wrinkles and for treating and/or improving disorders resulted in damage to the skin appearance, including preventing and/or treating a situation of abnormal hair loss (baldness).

It should be noted that hydrophobic and hydrophilic particles used in the present invention have a diameter, preferably in the range from about 5 to about 150 nm. The gaseous material may be air, ozone, oxygen and/or neutral gas.

### Background of the invention

WO 03/049706 the disclosure of which is incorporated herein by reference, describes an emulsion comprising water, hydrophilic particles (such as, for example, SiO₂, Al₂O₃, TiO₂, Fe₂O₃ and MnO) and hydrophobic particles (such as, for example, oxide particles having surfaces coated with non-polar, hydrophobic groups), wherein the hydrophilic and hydrophobic particles form shells encapsulating a gas that is suspended in the water, said shells comprising an external layer of hydrophilic particles and an internal layer of hydrophobic particles adjacent to the layer of hydrophilic particles.
WO 03/049706 further describes a powder comprising water, hydrophilic particles and hydrophobic particles, wherein the water is encapsulated in shells comprising an external layer of hydrophobic particles and an internal layer of hydrophilic particles adjacent to the layer of hydrophobic particles.
The concentration of hydrophobic particles in the composition described in WO 03/049706 determines its physical form, namely, in a relatively low concentration the composition is in an emulsion form, whereas in a relatively high concentration the composition is in a powder form. However, both emulsion and powder forms described in WO 03/049706 represent a mono-phase state since the emulsion form contains bounded shells encapsulating a gaseous material, and the powder form contains bounded shells encapsulating water. Neither form contains both mentioned bounded shells, side by side, simultaneously.

US Patent 6,808,715, the disclosure of which is incorporated herein by reference, describes an emulsion comprising water, hydrophilic particles comprising oxide particles having hydrophilic polar groups on their surface (such as, for example, -OH, CaCO₃, CuSO₄ and CaSO₄), and hydrophobic particles, wherein the hydrophilic and hydrophobic particles form shells as described in WO 03/049706.
The emulsions described in the above mentioned references are useful in preparation of topical cosmetic compositions for concealing wrinkles and for treating and/or improving disorders resulted in damage to the skin appearance.
Adding an insoluble, crystalline, solid polar ionic compound, such as CaCO₃, CuSO₄ and/or CaSO₄, to the hydrophilic particles, resulted in increasing and facilitating the contact between the hydrophilic particles that are located externally to the shells in the emulsion and the skin. Thus, the various ingredients of the cosmetic composition when applied to the skin form a direct contact with the skin surface as a consequence of the presence of polar crystalline structure bound to the external layer of the shell.
When applied onto the skin, the above described emulsions and powders may sometimes suffer from losing of too-much water in a relatively short time resulted in a rapid dryness. This dryness phenomenon resulted in reducing the effectiveness of the topical cosmetic composition in concealing wrinkles and in treating and/or improving disorders leading to damage to skin appearance. The relatively large evaporative surface area of the nanoparticles plays a significant role in increasing water evaporation, even at the storage state, resulted in shortening the composition's shelf-life. Consequently, for preparing a desired cosmetic composition having both, longer shelf-life and prolonged operation time, some additional means are required for increasing and/or stabilizing the attraction forces between the hydrophobic particles and water and between the hydrophobic particles and hydrophilic particles, when the latter are present. Such means should reduce the loss of water from said cosmetic composition.
It was surprisingly found that addition of a soluble electrolytic component, capable of releasing free ions in an aqueous environment, referred herein to as an electrolyte, to the emulsions and powders described hereinabove, resulted in production of a stable, prolonged release cosmetic composition that shows a substantial improvement in concealing wrinkles and in treating disorders leading to damage in skin appearance, including abnormal hair loss (baldness). The role of the electrolyte in the composition of present invention is to provide free ions for increasing the electrostatic and ionic forces between the water and the hydrophobic and hydrophilic particles, resulted in reducing the loss of water phenomenon. Furthermore, it was found that in many cases, the added electrolyte, may substitute the hydrophilic particles. In such cases, hydrophobic particles attract negative ions from the aqueous environment resulted in creating a water-diffusible layer on the surface of the hydrophobic particles. Substitution of hydrophilic particles by electrolyte means that no water in the prepared powder is bound to hydrophilic particles to form a gel-like structure. Consequently, a substantial increase in the amount of "free" water in the powder forms is obtainable as a result of using electrolyte for substituting hydrophilic particles, according to the present invention. Thus, a colloid-powder composition comprising water, hydrophobic particles, preferably having a diameter in the range of 5-150nm, and an electrolyte is formed, according to the present invention, and is very useful in preparing a fully active cosmetic composition formulated for concealing wrinkles and for treating and/or improving other disorders in the skin appearance, including abnormal hair loss (baldness). It should be emphasized that skin-care ingredients dispersed in "free" water show higher activity and effectiveness compared to the same ingredients when dispersed in bounded water, having ice-like structure with low molecular mobility.
The electrolyte in the composition of present invention plays a key role in the creation of a bi-phase state, namely the presence of bounded shells encapsulating a gaseous material side by side with bounded shells encapsulating aqueous solution droplets. The proportion of each type of shell in the composition is dependable by the concentrations of hydrophobic and hydrophilic particles as well as the electrolyte. Consequently, it is possible to monitor the amount of each type of shell by controlling the concentration of composition's particles and electrolyte.
It should be pointed out that when the composition of present invention comprises hydrophobic particles and electrolyte (no hydrophilic particles are present) it is considered a mono-phase composition having only one type of bounded shells, namely shells that encapsulate aqueous solution droplets. The addition of hydrophilic particles to the composition resulted in the formation of a bi-phase state in which both types of shells (the one that encapsulates a gaseous material and the one that encapsulates aqueous solution droplets) are present. The electrolyte in the composition of present invention provides free soluble ions that strengthen the links between water, hydrophilic and hydrophobic particles resulted in the stabilization of both types of shells presented in the bi-phase state.
The use of electrolyte in an emulsion and/or powder forms of composition, according to the present invention, provides a prolonged release, highly effective cosmetic composition having increased shelf-life. In addition, in many cases, the presence of electrolytes in the cosmetic composition avoids the need of addition of other stabilizing and/or preservative agents.

A metal oxide hydrophilic particle, such as, for example Al₂O₃, TiO₂, Fe₂O₃ or MnO is considered a relatively more positively-charged particle, compared to the non-metal oxide hydrophilic particle, such as, for example, SiO₂. More specifically, the positively-charged metal of the metal oxide hydrophilic particle may attract negatively-charged ions, whereas the negatively-charged oxygen of both, metal and non-metal oxide hydrophilic particles may attract positively-charged ions present in the medium. Consequently, a combination of metal and non-metal oxide hydrophilic particles in the composition of present invention is correspondingly resulted in association of the particles with both negatively- and positively-charged ions present in the medium.

It is known that addition of increasing amounts of electrolyte to water resulted in shifting pH values towards the alkaline range (pH>7). In view of this fact, it was surprisingly found that adding increasing amounts of electrolyte, such as, for example, NaCl to a system comprising water, hydrophobic particles, such as, for example, hydrophobic silica nanoparticles and hydrophilic particles, such as, for example, hydrophilic silica nanoparticles retains the pH of the system on the acidic side (pH<7). More specifically, one would predict that addition of increasing amounts of electrolyte to such system will result in dramatically changes in pH values, from the acidic side (pH<7) to the alkaline side (pH>7). However, the presence of hydrophilic particles in the composition of present invention resulted in producing a buffering environment that allows insertion of high concentrations of electrolyte without significantly affecting the pH. Taking into consideration that the electrolyte in the composition of present invention plays an important role in stabilizing the composition and enhancing its biological effectiveness, this pH buffering capability has a great advantage in preparing cosmetic compositions, which contain high concentrations of electrolyte.

### Summary of the Invention

It is an object of present invention to provide a stabilized, prolonged release highly biologically active topical cosmetic composition formulated for concealing wrinkles and for treating and/or improving disorders resulted in damage to the skin appearance, including preventing and/or treating a situation of abnormal hair loss (baldness).

It is a further object of present invention to provide a composition comprising water, optionally containing 25 - 400ppm of Ag, hydrophobic particles, preferably having a diameter, ranged from about 5 to about 150 nm and/or hydrophilic particles preferably having a diameter, ranged from about 5 to about 150 nm and a soluble electrolytic component, capable of releasing free ions in an aqueous environment, referred to as an electrolyte, wherein when the concentration of hydrophilic particles is 0wt% then the concentration of hydrophobic particles is >0wt% and when the concentration of hydrophobic particles is 0wt% then the concentration of hydrophilic particles is >0wt%.

It is a further object of present invention to provide a colloid-gel stable composition comprising water, optionally containing 25 - 400ppm of Ag, hydrophilic particles, preferably having a diameter in the range of 5-150 nm, and an electrolyte.

It is a further object of present invention to provide a stable, colloid-powder composition that comprises water, optionally containing 25 - 400ppm of Ag, hydrophobic particles, preferably having a diameter in the range of 5-150nm, and an electrolyte, wherein the hydrophobic particles bounding shells that encapsulate aqueous solution droplets.
The ions derived from the electrolytic component are associated with formation of strong electrostatic, ionic forces between the water and the hydrophobic particles, resulted in decreasing the amount of evaporable and/or removable free water from the composition. Consequently, such composition retains its water content to a longer period, avoiding any potential rapid dryness of the water encapsulated in the shells. Optionally, the hydrophobic particles may be partially, or entirely, coated by an amphiphilic substance (containing both hydrophilic and hydrophobic groups) to form a stable composition in which the water, soluble electrolyte and other effective skin-care components, such as, for example, essential oils, are located internally and externally to the shells encapsulated by a layer of the amphiphilicly-coated hydrophobic particles. Such composition of present invention may be highly effective in the treatment of a combination of disorders in skin appearance.

It is yet a further object of present invention to provide a bi-phase composition comprising water, optionally containing 25 - 400ppm of Ag, hydrophobic and hydrophilic particles, preferably having a diameter, ranged from about 5 to about 150nm, and an electrolyte, wherein such composition comprises simultaneously, bounded shells encapsulating a gaseous material, dispersed in water, side by side with bounded shells encapsulating aqueous solution droplets.
The electrolyte in such composition plays a key role in establishing the said unique bi-phase state. It releases ions that form strong electrostatic and ionic forces between the water and the hydrophobic particles and between the hydrophilic particles and hydrophobic particles. Consequently, the insertion of electrolyte into the composition, according to present invention, provides soluble free ions that function in stabilization of both types of shells existing in said bi-phase composition.
It is assumed that in this case the electrolyte stabilizes the shells of both the emulsion structure and the powder structure that exist in the bi-phase water-containing composition by increasing the affinity and the electrical attraction forces between the hydrophobic and hydrophilic particles and water.

It is yet a further object of present invention to provide a composition comprising water, optionally containing 25 - 400ppm of Ag, hydrophilic particles, preferably having a diameter, ranged from about 5 to about 150nm, an electrolyte, and an insoluble, crystalline, solid polar ionic compound, such as CaCO₃, CuSO₄ and/or CaSO₄, adsorbed on the surface of the hydrophilic particles. Hydrophobic particles, preferably having a diameter, ranged from about 5 to about 150nm, are optionally present in said composition.
The insoluble ionic compounds when adsorbed on the surface of a hydrophilic particle form a layer containing a large amount of sharp protrusions that increase and facilitate the contact between the hydrophilic particle and the skin. It further facilitates the direct contact of the various ingredients present in such cosmetic composition with the skin. It is estimated that these insoluble ionic compounds when adsorbed onto the surface of hydrophilic nanoparticle form a complex that provides a high electrical field having catalytic properties as well as strong polarization effect capable of attracting even non-polar substances present in the skin, such as, for example, fats and pus. The highly efficient anti-bacterial suppression effect of such composition of present invention should attributable to the formation of said complex when non-soluble ionic compounds are adsorbed on the surface of hydrophilic nanoparticle.

A further object of present invention is the preparation of a topical cosmetic formulation for concealing wrinkles and for treating and/or improving other disorders in skin appearance, such as, for example, acne, psoriasis, seborrhea, skin pigmentation, cellulitis, herpes, wound disinfection, skin fungus, skin irritation, allergies of the skin, eczema, atopic dermatitis, alopecia and warts, using any of the compositions discussed above.

### Description of the invention

The topical cosmetic composition, according to the present invention, comprises water, optionally containing 25 - 400ppm of Ag, hydrophobic particles, preferably having a diameter, ranged from about 5 to about 150 nm, and/or hydrophilic particles preferably having a diameter, ranged from about 5 to about 150 nm and an electrolyte, wherein when the concentration of hydrophilic particles is 0wt% then the concentration of hydrophobic particles is >0wt% and when the concentration of hydrophobic particles is 0wt% then the concentration of hydrophilic particles is >0wt%. This composition is specifically formulated for concealing wrinkles and for treating and/or improving other disorders resulted in damage to the skin appearance, including hair loss. Such topical composition, according to the present invention, may contain additional conventional and/or non-conventional ingredients beneficial for skin care, for example an oil, such as, sea buckthorn oil, primrose oil, almond oil and tea tree oil, vitamin A, beta carotene, vitamin E, vitamin C, anti-oxidants, anti-radicals, hydroxy-carboxylic acids, such as, for example octylhydroxy stearate, octylhydroxy acetate and salicylic acid, carboxylic acids, such as citric acid, propylene glycol, Fulvic acid, benzoyl peroxide, etc.

The topical cosmetic composition of the present invention may be in the form of lotion, cream, paste, ointment, gel, aerosol and powder.

The type, size, specific surface (in terms of m²/gr) and concentration of hydrophobic and hydrophilic particles in emulsion and powder forms of the composition of present invention are described in WO 03/049706 and US Patent 6,808,715. The gaseous material used in the emulsion of present invention is described in these references, as well.

The electrolyte used according to the present invention, may release in an aqueous environment a monovalent ion, a divalent ion, a multivalent ion or a mixture thereof.
A monovalent electrolyte may be represented by any soluble salt which releases free monovalent ions in aqueous environment, such as, for example, NaCl, KCl, LiCl, NaNO₃, CH₃COONa, CH₃COOAg, lactic acid salt.
A divalent electrolyte may be represented by any soluble salt which releases free divalent ions and/or a mixture of free divalent and monovalent ions in aqueous environment, such as, for example, CaCl₂, MgCl₂, Epsom salt (MgSO₄·7H₂O), Ca(NO₃)₂, (CH₃COO)₂Mg, ZnSO₄, (CH₃COO)₂Zn and a metal salt of dicaboxylic acids.
A trivalent electrolyte may be represented by any soluble salt which releases free trivalent ions and/or a mixture of free trivalent, divalent and monovalent ions in aqueous environment, such as, for example, FeCl₃ and metal salts of tricarboxylic acids.

Any mixture of electrolytes, such as, for example, the Dead Sea salt (comprising a mixture of MgCl₂ 33%, KCl 25%, NaCl 5.7% and CaCl₂ 0.3%) and any commercial biological active product containing salts or minerals that release ions in an aqueous environment, such as, for example, "Fulvic Acid Mineral Water" (by Springboard), is within the scope of present invention, as well.

An organic divalent- or multivalent ion [for example, the anions derived from di- and tri carboxylic acids, such as, succinic, gluconic, uric and citric acids and/or organic cations derived, for example, from di- and multi-amines and/or polar amphoteric molecule in a hybrid ions state (Zwitterion), for example an amino acid], form a linking bridge between similar and/or different hydrophobic nanoparticles, between hydrophobic and hydrophilic particles and between hydrophobic particles and water to yield a higher stabilized composition in either mono-phase or bi-phase state and consequently, provides a substantially more effective prolonged release topical cosmetic composition having longer shelf-life. Linking bridges may establish a well interconnected system showing a lower rate of water evaporation. More specifically, forming linking bridges may reduce the water evaporation by 10 to 100 fold. This reduction in evaporation plays an extremely important role in establishing the stable, long lasting, highly effective cosmetic composition of the present invention.

WO 03/049706 and US Patent 6,808,715 describe the various cosmetic applications of (1) emulsion which comprises water, hydrophobic and hydrophilic particles, preferably having a diameter in the range of 5-150 nm, wherein the hydrophobic and hydrophilic particles form shells encapsulating a gaseous material that is suspended in the water; said shells comprising an external layer of hydrophilic particles and an internal layer of hydrophobic particles adjacent to the layer of hydrophilic particles; and of (2) powder which comprises water, hydrophobic and hydrophilic particles, preferably having a diameter in the range of 5-150 nm, wherein the water is encapsulated in shells comprising an external layer of hydrophobic particles and an internal layer of hydrophilic particles adjacent to the layer of hydrophobic particles.
Using the cosmetic composition, according to the present invention, which comprises water, hydrophobic particles, preferably having a diameter, ranged from about 5 to about 150 nm, and/or hydrophilic particles preferably having a diameter, ranged from about 5 to about 150 nm and an electrolyte, wherein when the concentration of hydrophilic particles is 0wt% then the concentration of hydrophobic particles is >0wt% and when the concentration of hydrophobic particles is 0wt% then the concentration of hydrophilic particles is >0wt% may result in increasing, enhancing and facilitating one or more of the following biological effects associated with skin care activity:
- Reduction and/or removing wrinkles by straightening the wrinkled skin, flattening the furrows and making the wrinkles invisible and less pronounced. Flattering wrinkles furrows, reduces the pressure on blood capillaries network, resulted in restoring of normal blood supply.
- Skin reinforcement and rejuvenation:
   Due to the presence of electrolyte in the composition of present invention, it forms a stable layer when applied onto the skin surface. Said layer which consists of a stabilized nanoparticles network is strongly anchored to the skin and may be effective for several hours, if so desired. The relatively long lasting, stable network structure reinforces the treated skin area, inter alia, by unfolding the folded intracellular capillary vessels resulted in an enhancement and/or increasing of blood supply to the treated skin region, thus providing the important rejuvenation effect
- Skin complexion improvement:
   As was explained here-in-before, the presence of electrolyte in the composition of present invention, provides a stable, long lasting skin-care effective composition. When such composition is applied onto the skin surface, it forms a white, stabilized nanoparticles network which is strongly anchored to the skin and may stay in contact for several hours, if so desired. This white nanoparticles network provides a skin whitening effect, resulted in improving skin complexion.
- Moisturizing skin
- Antioxidant effect
- Skin cleaning (cleansers)
- Soft peeling:
   Soft peeling function of the composition of present invention resulted in removing impurities, scales and dead cells from treated skin surface, thus eliminating the favored natural environment required for fungi and bacteria growth.
- Bio-stimulation:
   The electrolyte present in the composition of present invention plays an important role in stimulating biological processes leading to renewal and/or recovery of damaged skin functions and/or appearance. The ions derived from the electrolyte are involved with and enhance a wide range of biological processes associated with the recovery of damaged skin, such as restoring capillary blood flow, cleansing, reinforcing and self-healing. In addition, said ions are associated with other skin care activities, such as:
   o Maintaining buffering environment for keeping unchanged the desired pH on the treated skin surface.
   **○** Redistributing electrolytes and/or ions between skin pores, capillaries, wounds and regular tissue surface to renew the desired net of typical periodic electric potential that is characteristic to normal healthy skin.
   **○** Maintaining electric polarization effect which accelerates skin cellular activity.
   o Facilitating the transformation of the composition of present invention, which consists of 3-dimensional colloidal nanoparticles system into a stable, long lasting, self-organized 2-dimentional network tightly anchored to the skin surface. Such transformation occurred when the composition is applied onto the skin surface and it is followed by the release into the skin of valuable skin-care ingredients. It should be mentioned that the ions present in the 2-dimensional network play a key role in controlling the release of skin-care ingredients and nutritional factors and in their penetration into the skin. Thus, the ions derived from the electrolyte play a significant role in the stabilization of the 2-dimensional network that covers and coats the skin. Consequently, they directly affect and control the prolonged release process of components and ingredients from the composition of present invention, when it is applied onto the skin surface.
- Anti-inflammatory effect.
- Soothing of irritated skin:
   The electrolyte which is present in the composition of present invention provides ions that upon application of the composition onto the skin surface produce a net of electric potentials causing the skin surface nerves to reduce their sensitivity. At the same time, some anti-inflammatory agents such as, for example, sea buckthorn oil and/or tea tree oil, are slowly released to provide long lasting skin soothing and anti-irritation effects. Thus, the composition of present invention when applied onto the skin surface produce a thin coating layer (which may be invisible, if so desired) which functions as a bandage having, inter alia, skin soothing and anti-irritation properties.
- Anti-ageing effect:
   The anti-oxidation activity of the composition of present invention, as well as its function in deactivation of free radicals play a critical role in forming a prominent anti-aging effect. More specifically, the composition found highly effective in removing from the treated skin zone dead cells, free radicals, toxins and other impurities having aging effect.

The cosmetic composition of present invention practically represents a structure consisting of a huge number of tiny particles, specifically arranged in aggregates, partially interconnected with soluble ions, which in the mono-phase state it contains either shells encapsulating gas and/or vaporized water (an emulsion structure or phase), or it contains shells encapsulating aqueous solution droplets (a powder structure or phase) and in the bi-phase state it contains both emulsion and powder structures (or phases), represented by the corresponding types of shells.

The electrolyte used according to the present invention plays a double role: (1) in stabilization of specifically designed emulsion or powder structure of the mono-phase cosmetic composition and in formation of the characteristic structure of bi-phase cosmetic composition which comprises simultaneously, side by side, both emulsion and powder structures; and (2) in facilitating and enhancing the favorable effects of ions on skin functions and appearance. Consequently, the cosmetic composition of present invention is highly effective as skin cleanser that removes dead cells and toxins from the skin nourishes and polishes it, and in many cases, repairs damage to skin functions and appearance originated from the accumulation of dead cells and/or toxins.
The electrolyte in the cosmetic composition of present invention is further associated in facilitating the important antioxidant effect of such composition.
It was further found that the presence of electrolyte in the cosmetic composition of present invention significantly accelerates the skin recovery from radiation damages that generate free radicals and cause burns and skin inflammations. It is assumed that the ions released from the electrolyte may restore electrical balance of damaged skin cells. Said ions may further act in a process of neutralization of free radicals and other harmful materials, such as toxins.

The cosmetic composition of present invention significantly accelerates the skin recovery process. For example, application of the composition for treating burns and/or following hair removal (inter alia by a laser based procedure) resulted in a full skin recovery within one week, while it usually takes 2-2.5 weeks. It is assumed that the composition of present invention functions as a buffer preventing further acidification of burns region and at the same time enhances capillary blood flow resulted in a recovery of damaged skin tissue.

The electrolyte used in the topical cosmetic composition of present invention, releases ions that play an important role in protecting the treated skin from bacterial and/or viral attack. In this respect, the cosmetic composition of present invention may be considered as an antiviral, anti-bacterial composition.

WO 03/049706 and US Patent 6,808,715 describe the mode of operation of a cosmetic composition consisting of an emulsion or powder comprising water, hydrophobic and hydrophilic particles. More specifically, these references relate to the mode of operation of said cosmetic composition which when applied onto the skin surface forms a layer consisting of network of filaments made of a particular combination of hydrophilic and hydrophobic particles. A portion of the hydrophilic particles migrates towards the sweat gland ducts located in the skin, forming tendrils of hydrophilic particles that penetrate into the ducts. A portion of the hydrophobic particles migrates and penetrates the ducts of sebaceous glands located in hair follicles inside the skin, forming tendrils of hydrophobic particles that protrude into the hair follicles and ducts of corresponding sebaceous glands. In particular, the hydrophilic and hydrophobic tendrils protrude into ducts of sweat and sebaceous glands that are located in furrows of wrinkles in the treated skin region.
Applying the cosmetic composition of present invention for reduction and/or removing wrinkles comprising the steps of straightening the wrinkled skin, flattening the furrows and making the wrinkles invisible or less pronounced. More particularly, the cosmetic composition when applied onto the skin surface forms a layer coating the region containing furrows of wrinkles. Such layer consists of network of filaments that are made of hydrophobic and/or hydrophilic particles. The network which is strongly anchored onto the skin surface covers the furrows of wrinkles.

The conversion of three-dimensional composition structure into two-dimensional structure is followed by breaking and opening the shells resulted in releasing gas and water, by means of evaporation and/or water flow into the skin. This process is followed by increasing attraction forces between particles constructing the network filaments resulted in contraction of filaments and subsequently the entire network.
The contraction of the network filaments resulted, inter alia, in flattening the furrows of wrinkles, making them invisible or less pronounced. It is speculated that during the contraction, the furrows of wrinkles are mechanically pulled out, thereby smoothing the skin and drawing the skin taut. The presence of an electrolyte in the cosmetic composition of present invention is resulted in the formation of stronger links between the same and/or different hydrophobic particles, between hydrophobic particles and the surrounding water, between the same and/or different hydrophilic particles, between hydrophobic particles and hydrophilic particles and between hydrophilic particles and the surrounding water. Consequently, a controlled slow-release effect of ingredients present in the cosmetic composition of present invention is obtainable.
The presence of a well controlled concentration of electrolyte in the cosmetic composition of the present invention, leads to the formation of two major types of compositions:
1. A hypotonic composition which directs the free water to flow towards and inward the skin, resulted in skin hydration effect; and
2. A hypertonic composition which pumps the water outward of skin, resulted in a skin dehydration effect.

It is clear that hypotonic composition comprises a relatively low concentration of electrolyte, whereas the hypertonic composition comprises a relatively high concentration of electrolyte. The composition of present invention comprises electrolyte in a wide range of concentrations, for example, from >0wt% to about 30wt% and preferably from 0.1wt% to about 20wt%. The concentration of electrolyte in hypotonic cosmetic composition is ranged from >0wt% to about 1wt% and preferably from about 0.1wt% to about 0.5wt% and it is dependable on the electrolyte used. For example, for NaCl, it is ranged from a concentration of >0wt% to a concentration of about 0.7wt% and preferably, from 0.1wt% to about 0.5 wt%. For ZnSO₄ it is from >0wt% to about 0.6wt% and preferably, from about 0.1wt% to about 0.4wt%. The concentration of electrolyte in hypertonic cosmetic composition is >1wt%, preferably from about 2wt% to about 20wt% and most preferably, from about 13wt% to about 20wt% when sodium chloride is used and from about 8wt% to about 20wt% when zinc sulphate is used. In terms of molar concentrations the hypotonic composition may comprise from >0M to about 0.1M sodium and potassium salts and hypertonic composition may comprise from about 0.15M to about 3.5M of the same salts.

Hypotonic compositions of present invention play an important role, for example, in peeling off dead skin cells from the epidermis. As a result of hypotonic concentration of electrolyte, water, ingredients and particles are moved from the cosmetic composition into the treated skin area to penetrate between dead skin cells and the surface of the epidermis.
In case of wet skin surface, water and predominantly hydrophilic particles intend to penetrate and accumulate between the dead skin cells and the epidermis. In case of oily skin surface, water and predominantly hydrophobic particles intend to penetrate and accumulate between the dead skin cells and the epidermis. When the dead skin cells are dry, water from the cosmetic composition will flow into the dead cells causing them to swell. The penetration of water into the dead cells and accumulation of the hydrophobic and/or hydrophilic particles between swelled dead skin cells and the epidermis resulted in mechanical pushing out and dislodging the dead skin cells from the epidermis. It should be emphasized that swelling dry dead skin cells by a hypotonic cosmetic composition, as itself, plays an important role in mechanically dislodging dead skin cells from the epidermis. The removal of dislodged dead skin cells from the skin, provides a soft peeling effect resulted in a significant improvement in skin elasticity and appearance.
Hypertonic cosmetic composition, according to the present invention, is useful, for example, in removing toxins and/or other undesired soluble materials accumulated in the interstitial fluids. When such cosmetic composition is in contact with the skin surface, liquids are moving from interstitial routes in the skin outwardly towards the applied cosmetic composition. The moved liquids may contain toxins and/or other solved undesired materials which eventually removed from the skin surface.

The composition of present invention may be useful in treatment of skin disorders, such as, for example, acne, psoriasis, seborrhea, skin pigmentation, cellulites, herpes, wound disinfection, skin fungus, skin irritation, allergies of the skin, eczema, atopic dermatitis, alopecia and warts.
In particular, it was found highly effective in treating oily, acne-prone skin due its prominent role in elimination of secondary infections, softening the skin, peeling the contaminated epidermis and improving blood circulation. It is further highly active in preventing or treating seborrhea, in absorbing infected secretions and drying up pimples. Acne is a product of inflamed sebaceous glands forming a plaque of fatty materials that prevent free flow of fluids. When the composition of present invention is applied onto the acne region, it provides ions associated-hydrophobic and hydrophilic (when present) nanoparticles that may favorably affect the damaged skin - as follows:
(1) Opening and/or removing the plaque of fatty materials from follicles (pores) to allow free flow of sebum oil to the surface. More specifically, in view of the high affinity exists between ions associated-hydrophobic nanoparticles provided by the composition of present invention and the fatty materials that form the plaque, the later are removed off the plaque. As a result, the plaque disintegrated and disappeared. Consequently, this operation keeps the follicles (pores) open and prevents the bacterial growth (resulted in inflammation) and subsequently the development of whiteheads and blackheads on the skin surface.
(2) The ions associated-hydrophobic nanoparticles provided by the composition of present invention may function in exchanging ions associated with fatty materials, resulted in inflammation suppression. Acne treatment may combine a procedure applying both, hypotonic and hypertonic compositions of the present invention. Applying hypotonic composition onto the acne zone causes flow of water from the composition towards and inwards the treated skin resulted in softening and moisturizing the treated acne zone. This is followed by the expansion of skin cells. The hypertonic composition that apply to the acne zone successively, causes a flow of water from the treated skin towards the composition layer removing from the treated acne zone dead cells, toxins and other undesired impurities. This sequential procedure (namely, successive applications of hypotonic and hypertonic compositions of present invention) may be repeated several times for better draining of sebaceous glands and preventing further development of inflammation. It should be noted that this procedure is effective for opening and cleaning both, the blackheads and whiteheads. The electrolyte present in the composition of present invention reduces the comedones oil's melting point and thus plays a key role in decreasing its viscosity.

It should be emphasized that the composition of present invention may be used for restoring the natural body healing resources in a procedure of skin treatment following disorders such as, canker sores, hives, picking, rosacea, scratching, shingles, vitiligo, warts, burns, dermatoses, itching and cholestasis.

### Description of the figures

Fig. 1 represents a graph of actual composition's density in an increasing concentration of electrolyte.
   Measured density values are represented by points.
   The bi-phase composition comprises 2.5wt% hydrophobic silica nanoparticles, 7.5wt% hydrophilic silica nanoparticles and a variable concentration of Dead Sea salt (0-30wt%).
Fig. 2 represents a graph of actual composition's porosity in an increasing concentration of electrolyte. The bi-phase composition is as described in Fig. 1.
Fig. 3 represents a graph of actual composition's pH in an increasing concentration of electrolyte.
   Measured pH values are represented by points.
   The bi-phase composition is as described in Fig. 1.
Fig.4 represents a graph of actual saline solution's pH in an increasing concentration of Dead Sea salt.
Fig. 5 represents a graph of actual composition's density in an increasing concentration of electrolyte.
   Measured density values are represented by points.
   The bi-phase composition comprises 5wt% hydrophobic silica nanoparticles, 5wt% hydrophilic silica nanoparticles and a variable concentration of Dead Sea salt (0-30wt%).
Fig. 6 represents a graph of actual composition's porosity in an increasing concentration of electrolyte. The bi-phase composition is as described in Fig. 5.
Fig. 7 represents a graph of actual composition's pH in an increasing concentration of electrolyte. The bi-phase composition is as described in Fig. 5.
Fig. 8 represents a graph of actual composition's density in an increasing concentration of electrolyte.
   Measured density values are represented by points.
   The mono-phase composition comprises 5wt% hydrophobic silica nanoparticles, 0wt% hydrophilic silica nanoparticles and a variable concentration of Dead Sea salt (0-30wt%).
Fig. 9 represents a graph of actual composition's pH in an increasing concentration of electrolyte. The mono-phase composition is as described in Fig. 8.

### Examples

### Example 1

The composition's density and porosity are dependable on the concentration of the electrolyte.
For determination of composition's density it is necessary to calculate the bulk density of the composition (gas pores are not included). In the first step, the density of the saline solution is determined. This can be done experimentally - as follows:
Dead Sea salt (a commercial product comprising: MgCl₂ 33%, KCl 25%, NaCl 5.7%, CaCl₂ 0.3% and water 36%), for example, is dissolved in pure water and the solution's density (g/ml) is determined for a range of salt concentrations from 0 to 30 wt%. There is a linear correlation between saline solution density and the different concentrations of inserted salt.

The saline solution density is determined using the following regression Equation:
Saline Solution Density (SSD) in g/ml = 1+3.257x10⁻³ C
wherein C represents salt concentration (wt%).

For describing the determination of bulk density of the composition of present invention, a series of compositions comprising 2.5 - 5% (by composition wt) of silica hydrophobic nanoparticles and 0 - 7.5% (by composition wt) of hydrophilic nanoparticles suspended in water containing Dead Sea salts in concentrations ranged from 0 to about 30% of water wt.

For the sake of demonstration, a reference is made to a 100g composition containing 90g of saline solution and total of 10g of hydrophobic and hydrophilic nanoparticles.
Taking into account that bulk density of silica is 2.65 g/ml, it is possible to calculate the bulk volume of silica:
Bulk volume of silica (ml) =10g/2.65g/ml = 3.774 ml
Saline solution volume can be calculated, as well:
Saline solution volume (ml) = 90g/SSD g/ml
wherein SSD stands for saline solution density.

The total volume of liquid and bulk (excluding gas pores) in the composition is calculated by the equation:
Total volume of liquid and bulk (ml) = 90g/SSD + 3.774

Consequently, the bulk density of the composition can be calculated:
Bulk density (g/ml) = 100g/Total volume of liquid and bulk

It should be pointed out that in this case there is a linear correlation between composition's bulk density and the different concentrations of inserted salt.

The porosity of the composition of present invention can be calculated, as follows:
Porosity = 1 - Actual density/Bulk density

The following examples deal with the determination of actual density and porosity of a series of compositions:

### Case 1:

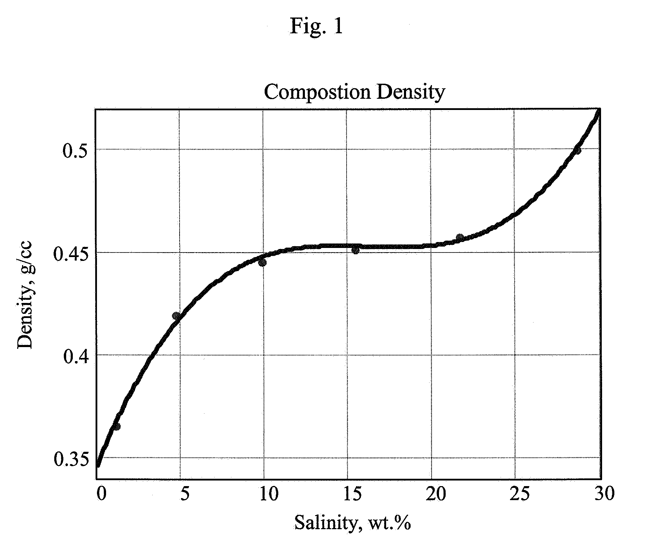

The composition comprises 2.5 wt% hydrophobic silica nanoparticles and 7.5 wt% hydrophilic silica nanoparticles.
This is a typical bi-phase composition that comprises both structures of shells: the emulsion structure (namely, the presence of bounded shells encapsulating gas, coated by an external layer of hydrophilic particles and an internal layer of hydrophobic particles) and the powder structure (namely, the presence of bounded shells encapsulating water, coated by an external layer of hydrophobic particles and an internal layer of hydrophilic particles). In addition, it comprises a relatively large amount of hydrophilic nanoparticles that are not associated with any shell structure, herein referred to as "free" hydrophilic particles. The proportion of shells of emulsion structure to the ones of the powder structure is dependable on concentration of electrolyte in the composition. The amount of hydrophilic particles that are not associated with shells ("free" hydrophilic particles) is about two-thirds of the total amount of hydrophilic nanoparticles.
Such a bi-phase composition that represents an excess of "free" hydrophilic nanoparticles shows some unique features, as shown in Figs. 1-3.

Fig. 1 represents the actual composition's density for each concentration of added salt up to 30wt%. The graph shows that a concentration of Dead Sea salt in the range between about 10wt% and about 25 wt% does not affect the density of the composition.
It is speculated that this is a typical effect of an electrolyte on the density of such bi-phase emulsion of present invention, having an excess of hydrophilic particles. In low concentrations of electrolyte (<10wt%), the free ions that are released in an aqueous environment function in strengthening the links between "free" hydrophilic particles and external layers of shells of either emulsion and/or powder structure on one side and between the nanoparticles layers that bound the same shell of either emulsion or powder structure, on the other. This ionic effect leads to a partial aggregation of shells, to reduction of distances between various shells and to some reduction in shells size, resulted in an increase in composition's density. Higher concentrations of salt (10 - 25wt%) function in stabilizing the ionic links with no significant effect on the density. Further increase of salt concentration (>25wt%) is resulted in an intensive aggregation of shells, in further reduction of distances between shells as well as in further reduction in shells size. This leads to a further increase in composition's density.

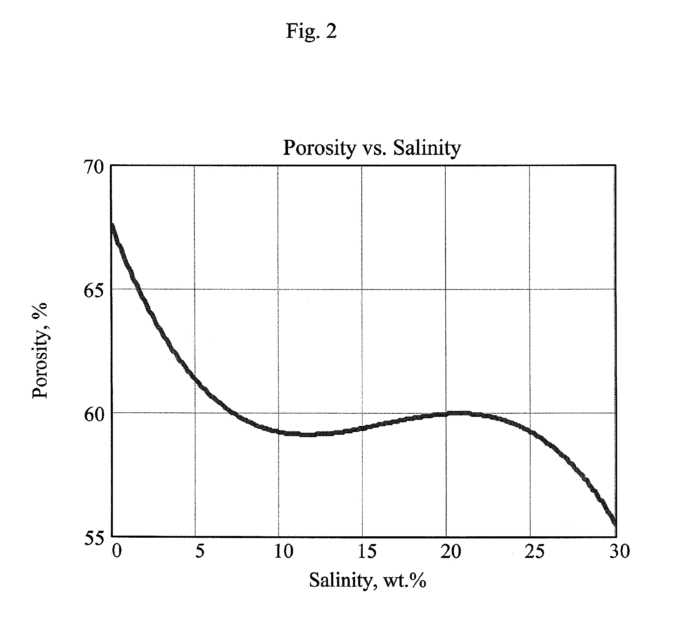

Fig. 2 represents the actual composition's porosity for each concentration of added salt up to 30wt%. The graph shows that a concentration of Dead Sea salt in the range between about 6wt% and about 25wt% does not affect the porosity of the composition.
It is speculated that changes in porosity, as well, are derived from same typical effect of an electrolyte on such bi-phase emulsion having an excess of hydrophilic nanoparticles. Low concentrations of electrolyte (<10wt%), lead to a partial aggregation of shells, to reduction of distances between shells and to some reduction in shells size. This is resulted in decreasing composition's porosity. Higher concentrations of salt (10 - 25wt%) function in stabilizing the ionic forces with no significant effect on the porosity.

Further increase of salt concentration (>25wt%) is resulted in an intensive aggregation of shells in further reduction of distances between shells and in further reduction in shells size. This leads to a further decrease in composition's porosity.

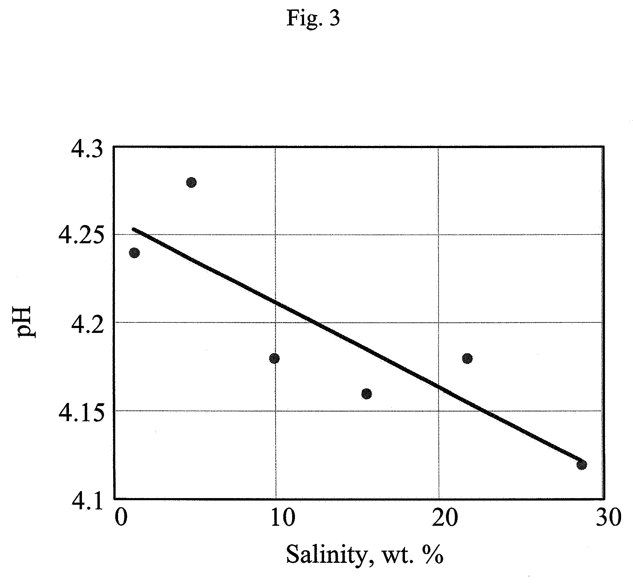

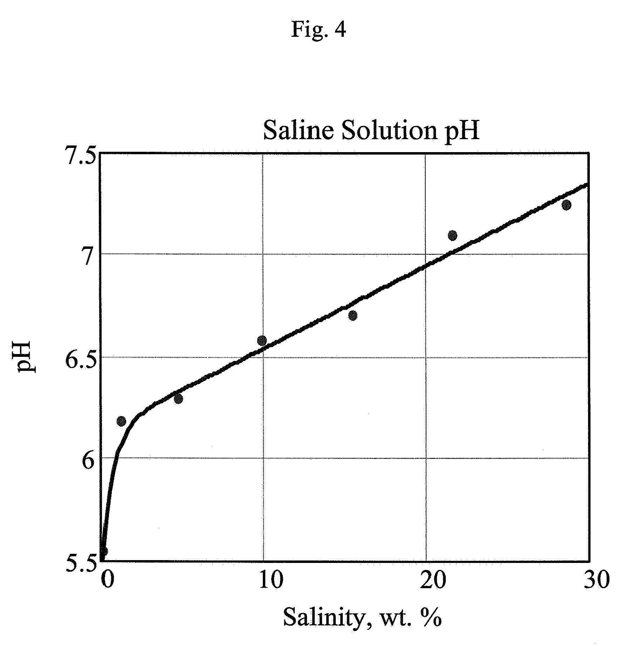

Fig. 3 represents the effect of salt concentration on the pH of same bi-phase composition. Addition of up to 30 wt% of Dead Sea salt resulted in slightly decrease in composition's pH values.
This is an unexpected finding in view of the fact that addition of Dead Sea salt to water resulted in an increase in pH values (cf. Fig. 4). It is speculated that hydrophilic particles in the bi-phase composition of present invention play a key role in formation of pH buffering effect. The excess of hydrophilic nanoparticles, in the bi-phase composition, as presented in this case, leads to formation of a strong pH buffering effect.

### Case 2:

The composition comprises 5wt% hydrophobic silica nanoparticles and 5 wt% hydrophilic silica nanoparticles.
This is another case of a bi-phase composition that comprises both structures of shells: the emulsion structure and the powder structure. In contrast to previous case (Case 1) the composition in this case does not comprise a significant amount of free hydrophilic nanoparticles that are not associated with any shell structure (only 10-30% of hydrophilic nanoparticles are free compared to about 67% in Case 1). The proportion of shells of emulsion structure to shells of powder structure is dependable on the concentration of electrolyte in the composition. For example, in low concentrations of salt the proportion of shells of emulsion structure to the ones of powder structure is 9:1, 1:1 and 1:4 in low, medium and high concentrations of salt, respectively.

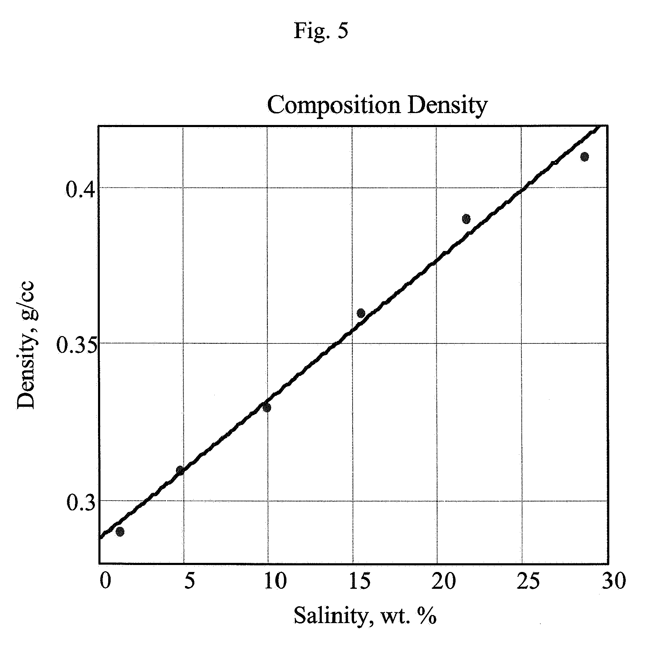

Such a bi-phase composition that comprises at least 70% of hydrophilic nanoparticles being in association with shells and represents either emulsion form (in a low concentration of salt) and/or powder form (in a high concentration of salt) shows different features, as shown in Figs. 5-7.

Fig. 5 represents the actual composition's density for each concentration of added salt up to 30wt%. The graph shows an ascending linear correlation between the actual composition's density and the different concentrations of inserted Dead Sea salt. The non-linear correlation shown in Fig. 1 (Case 1) reflects a bi-phase composition in which the amount of hydrophilic particles is in excess over the amount of hydrophobic particles. Such situation maintains a significant amount of "free" hydrophilic particles that are not associated with shells of either emulsion or powder structure. It is estimated that the presence in the bi-phase composition of a substantial amount of "free" hydrophilic particles plays a key role in the formation of non-linear correlation between composition's density and salt concentration.

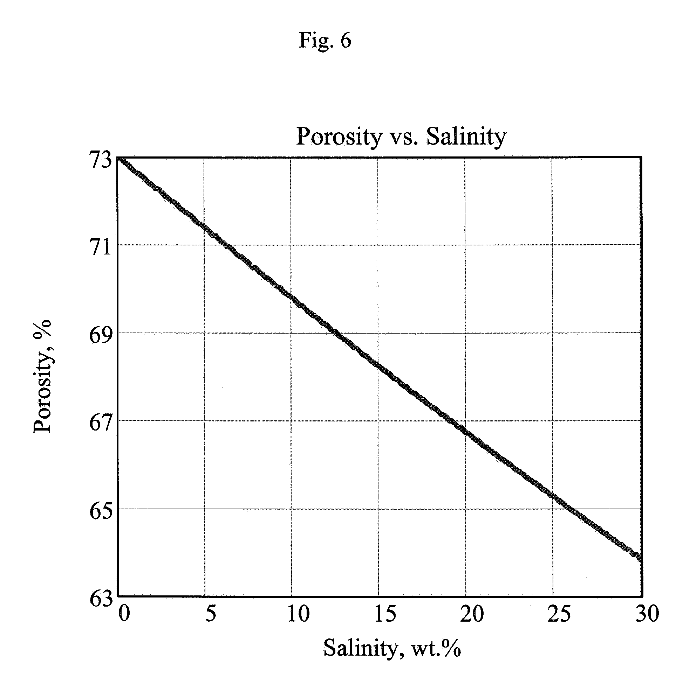

Fig. 6 represents the actual composition's porosity for each concentration of added salt up to 30wt%. The graph shows a descending linear correlation between the actual composition's porosity and the concentrations of inserted Dead Sea salt. The non-linear correlation shown in Fig. 2 (Case 1) reflects a bi-phase composition in which the amount of hydrophilic particles are in excess over the amount of hydrophobic particles. The discussion made in the description of Fig 5, is relevant to this figure, as well.

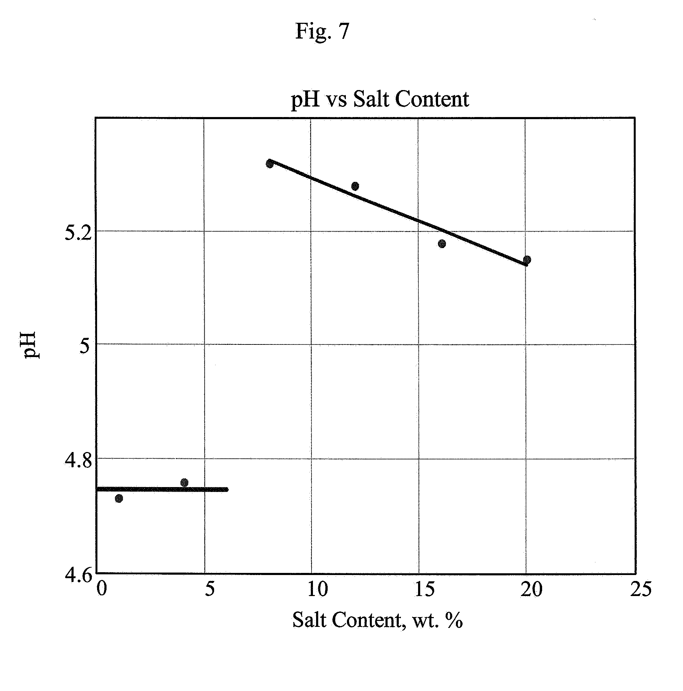

Fig. 7 represents the effect of salt concentration on the pH of the bi-phase composition. Addition of up to 5wt% of Dead Sea salt does not significantly affect the composition's pH values. The "jump" in pH values from about 4.7 to about 5.3 in a salt concentration of about 7wt% should be noted.
It is estimated that in low concentrations of salt the composition is mainly in an emulsion phase. It comprises very low amount, if at all, of shells of powder structure (or powder phase). Upon increasing the concentration of salt the composition is shifted to the powder form and the proportion of shells of powder phase is gradually increased. The shells of powder structure (or powder phase) appear when salt concentration becomes greater then a certain threshold (about 7wt% in present case). At that point, the shells of powder phase start to contribute to the measured pH values of the composition. Thus, a "jump" of the pH values occurs when the concentration of salt reaches the point in which it is capable of forming new shells of powder structure next to the already existing shells of emulsion structure (or phase).
There is an effect of slight decrease in pH values when salt concentrations are increased from about 10wt% to about 20wt%.
Here again, it is speculated that the presence of a sufficient amount of hydrophilic nanoparticles in a bi-phase composition, as presented in this case, plays a key role in the formation of pH buffering environment.

### Case 3

The composition comprises 5wt% hydrophobic silica nanoparticles and no hydrophilic silica nanoparticles.
This is a typical composition that solely comprises shells of a powder structure (or powder phase) coated by a layer of hydrophobic particles.
In the absence of hydrophilic particles, the composition in this case is in a "water powder" form. More specifically, the saline solution droplets are covered by a layer of hydrophobic silica nanoparticles. In view of absence of shells of emulsion structure the term porosity is not applicable in this particular case.

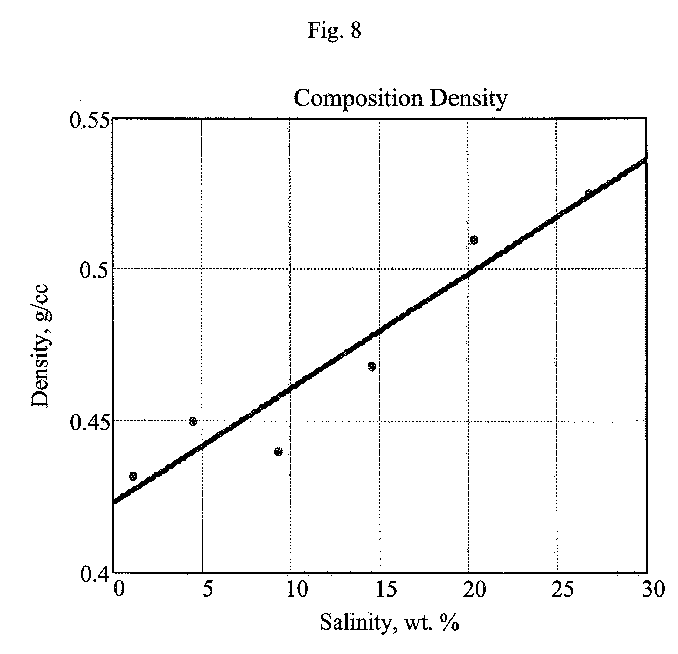

Fig. 8 represents the actual composition's density for each concentration of added salt up to 30wt%. The graph shows an ascending linear correlation between the actual composition's density and the concentrations of inserted Dead Sea salt. It is estimated that the free ions that are released in an aqueous environment function in strengthening the links between hydrophobic particles on one side and between hydrophobic particles and water on the other. This ionic effect is resulted in increasing aggregation of shells, in reduction of distances between shells and in reduction in shells size.

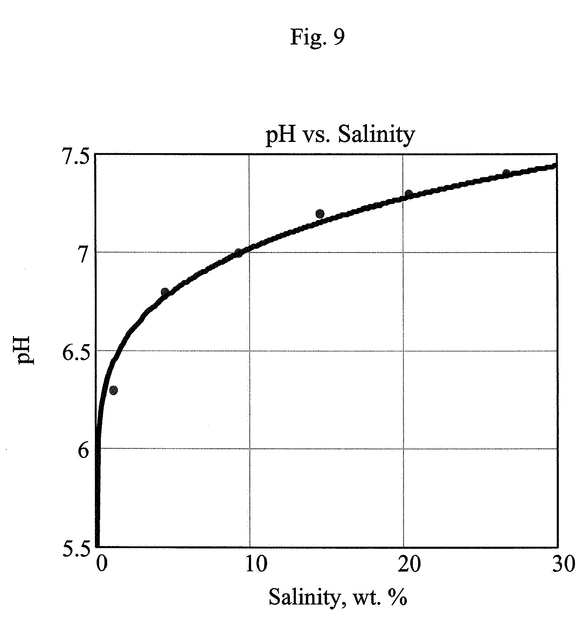

Fig. 9 represents the effect of salt concentration on the pH of the composition. An addition of about 10wt% of Dead Sea salt resulted in an increase of composition's pH to 7. Further increase in salt concentration (>10 wt%) resulted in pH values on the alkaline side. In the absence of hydrophilic particles the pH buffering effect of the composition is substantially reduced.

### Example 2

This example deals with the preparation of a composition comprising water, optionally containing 25 - 400ppm of Ag, 10wt% silica hydrophilic nanoparticles, 2.5wt% silica hydrophobic nanoparticles, Dead Sea salt (1 to 20wt%) and optionally additional conventional ingredients. 875g saline solution (containing 1 to 20wt% Dead Sea salt dissolved in purified water or dissolved in water containing Ag in an amount of 25-400ppm) and 50g Aerosil 380 (a commercial product of silica hydrophilic particles, having diameters in the range of 5-150nm, produced by Degussa of Germany) are mixed together for about 10 minutes in a mixer having a propeller rotating at about 1000rpm (rotations per minute). To the formed gel-like mixture, 25g Aexosil R812 (a commercial product of silica hydrophobic particles, having diameters in the range of 5-150nm, produced by Degussa of Germany) are added in three batches: each batch which contains about 8.3g is mixed for about 20 minutes at about 1000rpm. Following the addition of Aerosil R812, the mixture is further mixed for about 30 minutes at 1500-1800rpm. To the formed mixture, 50g Aerosil 380 are added and the mixture is mixed for about 30 minutes at 1000rpm. The formed mixture is set aside for a period of about 24 hours, during which it is maintained at a constant temperature of about 20°C and isolated from mechanical vibration and shock. Following this "maturation" period, the formed mixture is ready for optionally insertion (generally, by mixing for 10 minutes at about 500rpm) of one or more skin-care conventional ingredients such as, for example, evening primrose oil (EPO), sweet almond oil, sea buckthorn oil, tea tree oil, Finsolv TN(C₁₂ - C₁₅ alkyl benzoate), octyl hydroxystearate, salicylic acid, vitamin C, citric acid and benzoyl peroxide.
Air, ozone, oxygen or neutral gas may be suspended in water and encapsulated within the composition - as follows:
- Removing gas from purified water;
- While mixing the components during the formation of the composition, it should be hermetically sealed from the surrounding atmosphere. The hydrophilic and hydrophobic nanoparticles should be inserted into the electrolyte solution by suction with the chosen gas.

### Example 3

This example deals with a composition comprising water, optionally containing Ag in an amount of 25-400ppm, 10wt% Aerosil 380 (silica hydrophilic nanoparticle), 2.5wt% Aerosil R 812 (silica hydrophobic nanoparticles), Dead Sea salt (1 to 20wt%) and optionally one or more conventional skin-care and/or anti-acne agent, selected from the group consisting of evening primrose oil (EPO), sweet almond oil, sea buckthorn oil, tea tree oil, Finsolv TN (C₁₂ - C₁₅ alkyl benzoate), octyl hydroxystearate, salicylic acid, vitamin C, citric acid, azelaic acid, benzoyl peroxide, zinc acetate and sulphur. Said composition found to be highly effective in treating acne. It should be pointed out that the concentration of the salt in such composition is determined according to the treated skin type (dried, oily, etc) and the particular acne type, grade and state of the treated individual. Compositions containing higher concentrations of salt (10 - 20wt%) are preferred for treating an oily skin and an intensive acne state.

### Example 4

The composition which comprises water, optionally containing Ag in an amount of 25-400ppm, 5wt% Aerosil 380 (silica hydrophilic nanoparticle), 5wt% Aerosil R 812 (silica hydrophobic nanoparticles), Dead Sea salt (1 to 20wt%) and optionally one or more conventional skin-care and/or anti-acne agent, as described in Example 3 found to be highly effective in treating acne.

### Example 5

The composition which comprises water, optionally containing Ag in an amount of 25-400ppm, 5wt% Aerosil R 812 (silica hydrophobic nanoparticles), Dead Sea salt (1 to 20wt%) and optionally one or more conventional skin-care and/or anti-acne agent, as described in Example 3 found to be highly effective in treating acne.

### Example 6

This example deals with a composition comprising water, optionally containing Ag in an amount of 25-400ppm, 10wt% Aerosil 380 (silica hydrophilic nanoparticle), 2.5wt% Aerosil R 812 (silica hydrophobic nanoparticles), Dead Sea salt (1 to 20wt%) and optionally one or more conventional skin-care ingredient, selected from the group consisting of evening primrose oil (EPO), sweet almond oil, sea buckthorn oil, tea tree oil, borage oil, Finsolv TN (C₁₂ - C₁₅ alkyl benzoate), octyl hydroxystearate, salicylic acid, vitamin C and citric acid. Said composition found to be highly effective in concealing wrinkles and in treating disorders resulted in damage to the skin appearance. It should be pointed out that the concentration of the salt in such composition is determined according to the treated skin type (dried, oily, etc). Compositions containing higher concentrations of salt (10 - 20wt%) are preferred for treating an oily skin.

### Example 6a

The composition of Example 6 comprises 12wt% Dead Sea salt and 2wt% sweet almond oil.

### Example 6b

The composition of Example 6 comprises 16wt% Dead Sea salt and 3wt% evening primrose oil.

### Example 6c

The composition of Example 6 comprises 12wt% Dead Sea salt and 4wt% sea buckthorn oil.

### Example 6d

The composition of Example 6 comprises 8wt% Dead Sea salt and 3wt% Finsolv TN (C₁₂ - C₁₅ alkyl benzoate).

### Example 6e

The composition of Example 6 comprises 1wt% Dead Sea salt and 3wt% octyl hydroxylstearate.

### Example 7

This composition deals with a composition comprising water, optionally containing Ag in an amount of 25-400ppm, 5wt% Aerosil 380 (silica hydrophilic nanoparticle), 5wt% Aerosil R812 (silica hydrophobic nanoparticles), Dead Sea salt (1 to 20wt%) and optionally one or more conventional skin-care ingredient, selected from the group consisting of Evening primrose oil (EPO), Sweet almond oil, Sea buckthorn oil, Tea tree oil, borage oil, Finsolv TN (C₁₂ - C₁₅ alkyl benzoate), octyl hydroxystearate, salicylic acid, vitamin C and citric acid. Said composition found to be highly effective in concealing wrinkles and in treating disorders resulted in damage to the skin appearance. It should be pointed out that the concentration of the salt in such composition is determined according to the treated skin type (dried, oily, etc). Compositions containing higher concentrations of salt (10 - 20wt%) are preferred for treating an oily skin.

### Example 8

**(A). Hypotonic composition for treating acne**

| **Formulation in wt%** | |
|---|---|
| Dead Sea salt | 0.2 |
| Zinc sulfate | 1 |
| Hydrophobic silica | 5 |
| Hydrophilic silica | 5 |
| Tea tree oil | 2 |
| Sea buckthorn oil | 3 |
| Vitamin A | 0.1 |
| Vitamin C | 1.5 |
| Vitamin E acetate | 0.1 |
| Methylparaben | 0.1 |
| Propylene glycol | 2 |
| Purified water | 80% |

The water may optionally contain 25 - 400 ppm of Ag.
**(B). Hypertonic composition for treating acne**

| **Formulation in wt%:** | |
|---|---|
| Dead Sea salt | 14 |
| Zinc sulfate | 6 |
| Hydrophobic silica | 5 |
| Hydrophilic silica | 5 |
| Tea tree oil | 3 |
| Sea buckthorn oil | 5 |
| Vitamin A | 0.1 |
| Vitamin C | 1.7 |
| Vitamin E acetate | 0.1 |
| Methylparaben | 0.1 |
| Purified water | 60 |

The water may optionally contain 25 - 400 ppm of Ag.

### Example 9

This example deals with a composition applicable for preventing abnormal hair loss and for treating alopecia:

| **Formulation in wt%** | |
|---|---|
| Dead Sea salt | 0.5 |
| Hydrophilic silica | 4 |
| Hydrophobic silica | 6 |
| Propylene glycol | 30 |
| Methylparaben | 0.1 |
| Sea buckthorn oil | 2 |
| Minoxidil | 7.4 |
| Purified water | 50 |

The water may optionally contain 25 - 400 ppm of Ag.
This is a highly effective composition. In addition to its function in removing undesired fatty materials from the treated area it plays an important role in facilitating the penetration of minoxidil (an anti-balding drug that is applied directly to the scalp) inside the hair follicles.

### Example 10.

This example deals with a highly effective composition for treating eczema and atopic dermatitis:

| **Formulation in wt%** | |
|---|---|
| Hydrophilic silica | 7.5 |
| Hydrophobic silica | 2.5 |
| Dead Sea salt | 12 |
| Zinc acetate | 5 |
| Methylparaben | 0.1 |
| Retinyl palmilate | |
| (Vitamin A) | 0.1 |
| Aloe vera | 0.1 |
| Vitamin E acetate | 0.1 |
| Primrose oil | 3 |
| Sea buckthorn oil | 3 |
| Borage oil | 3.6 |
| Purified water | 63 |

The water may optionally contain 25 - 400 ppm of Ag.

### Example 12

**A composition for treating acne**

| **Formulation in wt%** | |
|---|---|
| Hydrophilic silica | 7.5 |
| Hydrophobic silica | 2.5 |
| Dead Sea salt | 20 |
| Propylene glycol | 3.0 |
| Retinyl palmilate | 0.10 |
| Methyl paraben | 0.20 |
| Propyl paraben | 0.10 |
| Aloe barbadensis (Aloe vera) leaf powder | 0.10 |
| Tocopheryl acetate (Vitamin E) | 0.10 |
| Salicylic acid | 0.5 to 2.0 |
| Purified water | 64.4 to 65.9 (depending on the amount of salicylic acid) |

| | |
|---|---|
| Note:Similar formulations for treating acne containing higher concentrations of salicylic acid (such as, for example, 3 to 12wt%) are within the scope of present invention, as well. | |

### Example 13

**A composition in the form of cream for treating cellulitis**

| **Formulation in wt%** | |
|---|---|
| Hydrophilic silica | 7.5 |
| Hydrophobic silica | 2.5 |
| Dead Sea salt | 18.7 |
| Propylene glycol | 2.8 |
| Retinyl palmilate | 0.09 |
| Methyl paraben | 0.18 |
| Propyl paraben | 0.09 |
| Aloe barbadensis (Aloe vera) leaf powder | 0.09 |
| Tocopheryl acetate (Vitamin E) | 0.09 |
| Imadazoidinyl urea | 0.18 |
| Kojic acid | 0.5 |
| Limon oil | 1.0 |
| Wheat germ oil | 1.0 |
| Joroba oil | 1.0 |
| Peach oil | 0.5 |
| Calendula oil | 0.5 |
| Eucalypt oil | 0.5 |
| Sea buckthorn | 0.5 |
| White clay | 1.0 |
| Purified water | 61.28 |

## Claims

1. A composition comprising water, optionally containing 25 - 400 ppm of Ag, hydrophobic particles, preferably having a diameter, ranged from about 5 to about 150nm, and/or hydrophilic particles preferably having a diameter, ranged from about 5 to about 150 nm and a soluble electrolytic component, capable of releasing free ions in an aqueous environment, referred herein to as an electrolyte, wherein when the concentration of hydrophilic particles is 0wt% then the concentration of hydrophobic particles is >0wt% and when the concentration of hydrophobic particles is 0wt% then the concentration of hydrophilic particles is >0wt%.

2. A composition according to claim 1, in which both hydrophilic and hydrophobic particles are present, referred to as a bi-phase composition, wherein said composition simultaneously comprises bounded shells encapsulating a gaseous material, dispersed in water, side by side with bounded shells encapsulating aqueous solution droplets.

3. A composition according to claim 2, comprises bounded shells encapsulating a gaseous material, coated by an external layer of hydrophilic particles and an internal layer of hydrophobic particles, referred to as shells of emulsion structure or of emulsion phase, and bounded shells encapsulating aqueous solution droplets coated by an external layer of hydrophobic particles and an internal layer of hydrophilic particles, referred to as shells of powder structure or of powder phase.

4. A composition according to claim 1, in which hydrophilic particles are not present, comprising bounded shells encapsulating aqueous solution droplets coated by a layer of hydrophobic particles.

5. A composition according to claim 1, in which the concentration of hydrophobic particles is 0wt%.

6. A composition according to any of claims 1 to 5, wherein the electrolyte is capable of releasing monovalent ions.

7. A composition according to any of claims 1 to 5, wherein the electrolyte is capable of releasing divalent ions or a mixture of mono- and divalent ions.

8. A composition according to any of claims 1 to 5, wherein the electrolyte is capable of releasing trivalent ions or a mixture of mono- , di- and trivalent ions.

9. A composition according to any of claims 1 to 8, comprising a number greater than 1 of electrolytes.

10. A composition according to any of claims 1 to 9, comprising electrolyte in a concentration ranged from 0.1wt% to about 30wt%, preferably from 0.5wt% to 20wt%.

11. A composition according to claim 6, wherein the electrolyte is selected from the group consisting of NaCl and KCl.

12. A composition according to claim 7, wherein the electrolyte is selected from the group consisting of MgCl₂, CaCl₂, ZnSO₄ and zinc acetate.

13. A composition according to claim 8, wherein the electrolyte is FeCl₃.

14. A composition according to claim 9, wherein the electrolyte is a mixture of electrolytes comprising MgCl₂ 33%, KCl 25%, NaCl 5.7% and CaCl₂ 0.3%

15. A composition according to any of claims 1 to 5 comprising electrolyte in a concentration ranged from >0wt% to about 1wt%, and preferably from 0.1wt% to about 0.4wt%, referred to as hypotonic composition.

16. A composition according to any of claims 1 to 4 comprising electrolyte in a concentration greater than 1wt%, preferably from about 2wt% to about 20wt% and most preferably from about 8wt% to about 20wt%, referred to as hypertonic composition.

17. A composition according to claims 2, 3 and 5, wherein at least part of the hydrophilic particles are dispersed in the water and form with the water a gel-like structure having filaments of hydrophilic particles to which water molecules adhere.

18. A composition according to any of claims 1 to 4, wherein the shells have a characteristic diameter in a range from about 1 micrometer to about 20 micrometers.

19. A composition according to any of claims 1 to 5, wherein the particles have a characteristic diameter ranged from about 5nm to about 150nm.

20. A composition according to any of claims 1 to 5, wherein the particles have a characteristic specific surface greater than about 100m²/g.

21. A composition according to any of claims 1 to 3 and 5, wherein the hydrophilic particles are oxides, preferably selected from the group consisting of SiO₂, Al₂O₃, TiO₂, Fe₂O₃ and MnO particles.

22. A composition according to claim 21, wherein a non-soluble crystalline ionic compound, selected from the group consisting of Ca₂SO₄, CuSO₄ and CaCO₃, is adsorbed on the surface of the hydrophilic oxide particle.

23. A composition according to any of claims 1 to 4, wherein the hydrophobic particles are oxide particles having hydrophobic groups on their surface.

24. A composition according to any of claims 1 to 4, wherein the gaseous material is selected from the group comprising air, ozone, oxygen and neutral gas.

25. A composition according to any of claims 1 to 5, further comprising an ingredient or a mixture of ingredients useful in concealing wrinkles and in treating and/or improving disorders resulted in damage to the skin appearance, including preventing and/or treating abnormal hair loss (baldness).

26. A topical cosmetic formulation for concealing wrinkles and for treating and/or improving disorders resulted in damage to the skin appearance, including preventing and/or treating abnormal hair loss (baldness), comprising a composition according to any of claims 1 to 5.

27. A formulation according to claim 26, further comprising oil and/or ingredient or a mixture of ingredients beneficial for skin treatment and care.

28. A formulation according to claim 27, wherein the oil is selected from the group consisting of evening primrose oil (EPO), sweet almond oil, sea buckthorn oil, tea tree oil and borage oil.

29. A formulation according to claim 27, wherein the ingredient or a mixture of ingredients beneficial for skin treatment and care are selected from the group consisting of vitamin E acetate, retinyl palmilate, salicylic acid, sulphur, zinc acetate, essential oils, citric acid, uric acid, ascorbic acid and benzoyl peroxide.

30. A formulation according to any one of claims 26 to 29, comprising a hypotonic composition, as claimed in claim 15.

31. A formulation according to any one of claims 26 to 29, comprising a hypertonic composition, as claimed in claim 16.

32. A formulation according to claim 26, wherein the disorders resulted in damage to the skin appearance, are selected from the group consisting of acne, psoriasis, seborrhea, skin pigmentation, cellulites, herpes, wound disinfection, skin fungus, skin irritation, allergies of the skin, eczema, atopic dermatitis, alopecia and warts.

33. A formulation according to claim 32 for treating oily, acne-prone skin suitable for elimination of secondary infections, softening the skin, peeling the contaminated epidermis, treating and/or preventing seborrhea, absorbing infected secretions, drying up pimples and improving blood circulation.

34. A method for reducing and concealing wrinkling in a region of skin comprising:
- forming a layer of a formulated topical composition according to any of claims 26 to 29 on the region of skin surface intended for treatment;
- waiting a sufficient period of time so that a portion of the water from the formulated composition is absorbed by the treated skin region resulted in volume shrinking of said layer followed by transforming the layer into a network of filaments or strands on the treated skin region, which network is anchored to the skin by attraction of hydrophobic particles and hydrophilic particles, whenever they are present, to the skin and tends to contract as water is absorbed from the emulsion.

35. A method for treating and/or improving disorders resulted in damage to the skin appearance, comprising:
- forming a layer of a formulated topical hypotonic (or, if so desired, hypertonic) composition according to any of claims 30 and 31 on the region of skin surface intended for treatment;
- waiting a sufficient period of time until skin hydration (or dehydration) process is completed; optionally followed by
- removing of said layer from the skin surface and successively forming a layer of a formulated topical hypertonic (or hypotonic) composition on same skin region; and
- waiting a sufficient period of time until skin dehydration (or hydration) process is completed.

36. A method for treating a skin disorder selected from the group consisting of acne, psoriasis, seborrhea, skin pigmentation, cellulites, herpes, wound disinfection, skin fungus, skin irritation, allergies of the skin, eczema, atopic dermatitis, alopecia and warts, comprising:
- forming a layer of a formulated topical composition, as claimed in claim 32, on the region of skin surface intended for treatment;
- waiting a sufficient period of time so that a portion of the water from the formulated composition is absorbed by the treated skin region resulted in volume shrinking of said layer followed by transforming the layer into a network of filaments or strands on the treated skin region, which network is anchored to the skin by the attraction of hydrophobic particles and hydrophilic particles, whenever they are present, to the skin and tends to contract as water is absorbed from the emulsion.

37. A method for treating oily, acne-prone skin, comprising:
- forming a layer of a formulated topical composition, as claimed in claim 33, on the region of skin surface intended for treatment;
- waiting a sufficient period of time so that a portion of the water from the formulated composition is absorbed by the treated skin region resulted in volume shrinking of said layer followed by transforming the layer into a network of filaments or strands on the treated skin region, which network is anchored to the skin by attraction of hydrophobic particles and hydrophilic particles, whenever they are present, to the skin and tends to contract as water is absorbed from the emulsion.

38. A process for preparing a composition according to any of claims 1 to 3, omprising:
- forming a solution of water, optionally containing 25 - 400 ppm ofAg and an electrolyte;
- adding about half of the desired quantity of hydrophilic particles to the solution to form a mixture;
- adding the desired quantity of hydrophobic particles to the formed mixture
- adding about the second half of the desired quantity of hydrophilic particles to the formed mixture;
- causing the desired gaseous material to be present in the mixture as dispersed in water
- causing the dispersed gas to be encapsulated in shells of emulsion structure and at least part of the aqueous solution to be encapsulated in shells of powder structure.

39. A process for preparing a composition according to claim 4, comprising:
- forming a solution of water, optionally containing 25 - 400 ppm of Ag and an electrolyte;
- adding the desired quantity of hydrophobic particles to the solution to form a mixture;
- causing at least part of the aqueous solution to be encapsulated in shells coated by the hydrophobic particles.

40. A composition for treating acne as described in any one of the Examples 3 to 5, 8(A), 8(B) and 12.

41. A composition for reducing and concealing wrinkling as described in Examples 6, 6a to 6e and 7.

42. A composition for preventing abnormal hair loss and treating alopecia as described in Example 9.

43. A composition for treating eczema and atopic dermatitis as described in Example 10.

44. A topical cosmetic composition for treating and/or improving disorders resulted in damage to the skin appearance, substantially as described in the specification.
